# EUROPÄISCHE PATENTSCHRIFT

(11) **EP 0 874 014 B1**
(45) Veröffentlichungstag und Bekanntmachung des Hinweises auf die Patenterteilung: **24.03.2004**
(21) Anmeldenummer: 98107250.7
(22) Anmeldetag: 21.04.1998
(51) Int. Cl.: C08G 63/47, C08F 290/06, C08G 63/52, C08G 63/676, C08G 63/91, C09D 167/06

(54) **Verfahren zur Herstellung von Estern mit geringem Restsäuregehalt aus Alpha, Bêta-ethylenisch ungesättigten Carbonsäuren und Hydroxylgrupen-haltigen Polymerisaten**
Process for the production of esters of ethylenically unsaturated carboxylic acids and hydroxyl groups containing polymerisates, having a low level of residual acid
Procédé de production d'esters d'acides carboxyliques insaturés éthyléniques etpolymérisats contenant des groupes hydrxyle, ayant une teneur faible en acide résiduel

(30) Priorität: 21.04.1997 DE 19716686
(43) Veröffentlichungstag der Anmeldung: 28.10.1998
(73) Patentinhaber: BASF AKTIENGESELLSCHAFT, 67056 Ludwigshafen (DE)
(72) Erfinder: Königer, Rainer, Dr., 63329 Egelsbach (DE); Reich, Wolfgang, Dr., 67133 Maxdorf (DE); Beck, Erich, Dr., 68528 Ladenburg (DE); Wolf, Lothar, 02991 Torno (DE); Mnich, Johannes, 03229 Altdöbern (DE); Gruner, Helmut, 01987 Schwarzheide (DE)
(74) Vertreter: Kinzebach, Werner, Dr.

(56) Entgegenhaltungen:
- EP-A- 0 126 341
- DE-A- 3 106 570
- DE-A- 4 126 359

## Beschreibung

Die vorliegende Erfindung betrifft ein Verfahren zur Herstellung von Estern α,β-monoethylenisch ungesättigter Mono- und -Dicarbonsäuren mit Hydroxylgruppen-haltigen Polyethern oder Polyestern (im folgenden als Polykondensate bezeichnet), wobei man die Säure und das Polymerisat unter Entfernung des dabei entstehenden Wassers zu einem Ester umsetzt und die restliche Säure destillativ abtrennt.

Ester ungesättigter Carbonsäuren mit Hydroxylgruppen-haltigen Polykondensaten haben z. B. in Form der Polyester- und Polyetheracrylate und -methacrylate eine große technische Bedeutung erlangt und breite Anwendung gefunden. Aufgrund ihrer Vernetzbarkeit bei Belichtung eignen sie sich vor allem zur Herstellung strahlungshärtbarer Überzugsmassen, z. B. von Lack-Formulierungen, welche durch UV- bzw. Elektronenstrahlen schnell gehärtet werden können. In Abhängigkeit vom Verwendungszweck der Ester ist eine Verringerung des Restsäuregehaltes erforderlich, da die eingesetzten α,β-ungesättigten Mono- und/oder -Dicarbonsäuren allergologisch und toxikologisch nicht ganz unbedenklich sind und evtl. auch zu einer Geruchsbelästigung führen können. Insbesondere bei einer Innenanwendung als Lacke z. B. auf Holzmöbeln ist ein geringer Säuregehalt anzustreben, da ansonsten Geruchsbelästigungen oder Hautreizungen von dem Möbelstück ausgehen könnten.

Die DE-A-33 16 593 beschreibt ein Verfahren zur Herstellung von Estern der (Meth)acrylsäure mit Hydroxylgruppen-haltigen Polyestern oder Polyethern, wobei die Veresterung in Gegenwart eines sauren Veresterungskatalysators und mindestens eines Kohlenwasserstoffes erfolgt, der mit Wasser ein azeotropes Gemisch bildet und als Schleppmittel für das bei der Veresterung entstehende und azeotrop abdestillierte Wasser dient. Im Anschluss an die Veresterung wird der Kohlenwasserstoff destillativ entfernt und der Veresterungskatalysator neutralisiert. Die Entfernung der restlichen Acryl- oder Methacrylsäure erfolgt durch Zugabe einer der Säurezahl des Polyester- oder Polyetheracrylats äquivalenten Menge einer Polyepoxidverbindung. Nachteilig an diesem Verfahren ist, dass nach der Veresterung unter azeotroper Entfernung von Wasser und nach der anschließenden Abtrennung des Schleppmittels immer noch Restmengen an Acrylsäure im Produkt enthalten sind, welche in einem zusätzlichen Verfahrensschritt durch Umsetzung mit dem Epoxid entfernt werden müssen. Die verlängerte Reaktorbelegzeit durch den zusätzlichen Reaktionsschritt und der Einsatz des relativ teuren Epoxids führen zu einer wirtschaftlichen Benachteiligung dieses Verfahrens.

Die DE-A-38 43 854 beschreibt ein Verfahren zur Herstellung von (Meth)acrylsäureestern mehrwertiger Alkohole durch Umsetzung der Reaktanten in Gegenwart von sauren Veresterungskatalysatoren unter Zusatz von Polymerisationsinhibitoren, wobei man weitgehend auf den Einsatz von Lösungs- und/oder azeotropen Schleppmitteln verzichtet, den Reaktionsraum mit einem sauerstoffhaltigen Gasstrom durchspült und das bei der Veresterung entstehende Kondensationswasser aus der Gasphase des Reaktionsraumes abzieht (Luftstrippen). Im Anschluss an die Umsetzung wird das den sauren Veresterungskatalysator enthaltende Reaktionsrohprodukt einer sogenannten trockenen Neutralisation durch Zusatz eines Neutralisationsmittels, wie z. B. eines Oxids und/oder Hydroxids der Alkalimetalle, der Erdalkalimetalle und/oder des Aluminiums unterzogen. Über den Restsäuregehalt der so erhaltenen Ester findet sich kein Hinweis. Die resultierenden Produkte sind jedoch häufig stark verfärbt und müssen einer abschließenden Behandlung mit Entfärbungsmitteln unterzogen werden.

Die EP-A-0 279 303 beschreibt strahlungshärtbare Acrylate, die durch gleichzeitige Umsetzung von mehrwertigen alkoxylierten Alkoholen mit einer 2- bis 4-wertigen C₃- bis C₃₆-Carbonsäure oder deren Anhydride und mit Acrylsäure und/oder Methacrylsäure erhältlich sind. Die Umsetzung erfolgt dabei in Gegenwart eines sauren Veresterungskatalysators und mindestens eines Kohlenwasserstoffs, der mit Wasser ein azeotropes Gemisch bildet sowie geringer Mengen eines Polymerisationsinhibitors unter azeotroper Entfernung des entstehenden Wassers. Nach destillativer Entfernung des Kohlenwasserstoffs und Neutralisation des Veresterungskatalysators werden die überschüssigen Carboxylgruppen mit einer äquivalenten Menge einer Epoxidverbindung umgesetzt.

Die DE-A-28 38 691 beschreibt durch Strahlung aushärtbare Acrylpolyester, Verfahren zu ihrer Herstellung, wobei man ein lineares Vorpolymerisat mit zwei endständigen Carboxylgruppen oder zwei endständigen Hydroxylgruppen mit wenigstens einer Dihydroxylverbindung bzw. einer Dicarbonsäure umsetzt und das resultierende Produkt anschließend mit Acrylsäure verestert. Zur Entfernung der überschüssigen Acrylsäure wird eine Lösung des Polyesters in einem organischen Lösungsmittel, wie z. B. Benzol, mehrmals mit wässrigen NaCl-Lösungen gewaschen und anschließend die organische Phase über wasserfreiem Natriumsulfat getrocknet und filtriert. Nachteilig an diesem Verfahren ist das Entstehen organisch belasteter Waschwässer und Trockenmittel, die aufwendig aufgearbeitet oder entsorgt werden müssen. Zudem führt wiederholtes Waschen und anschließendes Trocknen der Polyesteracrylat-haltigen Lösungen zu Produktverlusten.

Die DE-A-31 06 570 beschreibt (Meth)acryloyloxy-terminierte Polyester, die durch Umsetzung von Acryl- oder Methacrylsäure mit einem Hydroxy-terminierten Polyester hergestellt werden. Das resultierende Reaktionsprodukt kann durch Neutralisieren, Waschen mit Wasser, Entfärben und Filtrieren gereinigt werden.

Der vorliegenden Erfindung liegt die Aufgabe zugrunde, ein einfach durchführbares Verfahren zur Herstellung von Estern aus α,β-monoethylenisch ungesättigten Mono- und -Dicarbonsäuren und Hydroxylgruppen-haltigen Polykondensaten zur Verfügung zu stellen. Dabei sollen insbesondere die Nachteile der bisher üblichen Verfahren zur Entfernung der Restsäure durch einen zusätzlichen Reaktionsschritt, wie Umsetzung mit einem Epoxid oder durch technisch aufwendige Strip- und Waschverfahren vermieden werden. Das Qualitätsprofil der Ester soll im wesentlichen nicht beeinträchtigt werden, und vor allem sollen im wesentlichen die nach dem Stand der Technik möglichen, geringen Restsäuregehalte erzielt werden.

Überraschenderweise wurde nun gefunden, dass die Aufgabe gelöst wird, wenn man die unumgesetzte Säure destillativ entfernt und dabei Wasser zugibt. Dabei werden im wesentlichen die selben Restsäuregehalte wie bei den bisher üblichen Verfahren erreicht.

Gegenstand der Erfindung ist daher ein Verfahren zur Herstellung von Estern E mit geringem Restsäuregehalt aus α,β-monoethylenisch ungesättigten C₃-C₆-Mono- und Dicarbonsäuren S und Hydroxylgruppen-haltigen Polykondensaten P (insbesondere Polyether und Polyester), wobei man
a) die Säure S und das Polykondensat P oder die das Polykondensat P bildenden monomeren und/oder oligomeren und/oder polymeren Komponenten, gegebenenfalls in Gegenwart eines Lösungsmittels und/oder eines Veresterungskatalysators und/oder weiterer Zusatzstoffe, unter Entfernung des entstehenden Wassers umsetzt,
b) die restliche Säure S destillativ entfernt,
das dadurch gekennzeichnet ist, dass man zur destillativen Entfernung der Säure S in Schritt b) Wasser zugibt.

### Schritt a)

Die Veresterungsreaktion zwischen der α,β-monoethylenisch ungesättigten C₃-C₆-Mono- und -Dicarbonsäure S und den Hydroxylgruppen-haltigen Polykondensate P erfolgt nach allgemein bekannten Verfahren, wobei die Entfernung des gebildeten Reaktionswassers z. B. durch wasserentziehende Mittel, extraktiv oder azeotrop erfolgen kann. Vorzugsweise wird das gebildete Reaktionswasser azeotrop entfernt. Die Umsetzung erfolgt dabei in Gegenwart eines Lösungsmittels, das mit Wasser ein azeotropes Gemisch bildet. Geeignete Lösungs- und Schleppmittel sind aliphatische und aromatische Kohlenwasserstoffe, z. B. Alkane, wie n-Hexan und n-Heptan, Cycloalkane, wie Cyclohexan und Methylcyclohexan, Aromaten, wie Benzol, Toluol und Xylolisomere und sogenannte Spezialbenzine, welche Siedepunkte zwischen 70 und 140 °C aufweisen. Besonders bevorzugte Schleppmittel sind Cyclohexan, Methylcyclohexan und Toluol. Die Menge des Lösungsmittels kann für die azeotrope Entfernung des gebildeten Reaktionswasser in weiten Bereichen variiert werden. Geeignete Apparaturen zur azeotropen Destillation unter Abscheidung des Reaktionswassers und Rückführung des Lösungsmittels in das Reaktionsgefäß sind dem Fachmann bekannt. Das eingesetzte Lösungsmittel kann nach der Veresterung durch übliche Methoden, wie z. B. durch Destillation, gegebenenfalls unter vermindertem Druck, aus dem Reaktionsgemisch entfernt werden. Dabei wird vorteilhafterweise bereits ein Teil der überschüssigen Säure vom Reaktionsgemisch abgetrennt. Die Veresterungstemperatur liegt im allgemeinen in einem Bereich von etwa 60 bis 140 °C.

Bei der erfindungsgemäßen Veresterung liegt der Umsatz im allgemeinen bei mindestens 85 %, vorzugsweise bei 90 bis 95 %. Die Veresterung erfolgt im allgemeinen in Gegenwart eines Katalysators. Geeignete Katalysatoren sind starke Säuren, wie z. B. Schwefelsäure, wasserfreier Chlorwasserstoff, Sulfonsäuren, z. B. Toluolsulfonsäure und saure Ionenaustauscher. Im erfindungsgemäßen Verfahren wird vorzugsweise Schwefelsäure und p-Toluolsulfonsäure als Katalysator eingesetzt. Die Menge des Veresterungskatalysators liegt dabei im allgemeinen in einem Bereich von etwa 0,1 bis 5 Gew.-%, bezogen auf die Gesamtmenge an zu veresternden Komponenten.

Zur Vermeidung einer vorzeitigen Polymerisation erfolgt die Veresterung in Schritt a) vorzugsweise in Gegenwart eines Polymerisationsinhibitors. Geeignete Polymerisationsinhibitoren sind die üblichen, zur Verhinderung einer thermischen Polymerisation verwendeten Verbindungen, wie Derivate des Hydrochinons, bevorzugt Hydrochinonmonoalkylether, z. B. Hydrochinonmonomethylether, substituierte Phenole, z. B. p-Methoxyphenol, 2,5-Di-t.-butylp-kresol, 2,6-Di-t.-butylphenol, 2,6-Di-t.-butyl-4-methoxyphenol, 2-t.-Butyl-4-methoxyphenol, 3-t.-Butyl-4-methoxyphenol, 2,4,6-Tri-t.-butylphenol etc., t.-Butylbrenzkatechin, Derivate der N-Nitrosamine, Phenothiazin, Phosphorigsäureester, wie Triphenylphosphit, Diethylphosphit, Tridecylphosphit, Triisodecylphosphit etc., sowie hypophosphorige Säure. Sie werden im allgemeinen in Mengen von etwa 0,001 bis 2,0 Gew.-%, vorzugsweise etwa 0,005 bis 0,5 Gew.-%, bezogen auf die Gesamtmenge an zu veresternden Komponenten, eingesetzt.

Nach einer weiteren geeigneten Ausführungsform kann man zur Herstellung der Ester E die Säure S und die das Polykondensat P bildenden monomeren und/oder oligomeren und/oder polymeren Komponenten im Sinne einer einstufigen Synthese umsetzen. Die EP-A-0 279 303 beschreibt ein Verfahren zur Herstellung strahlungshärtbarer Acrylate durch gleichzeitige Umsetzung aller Edukte, worauf hiermit Bezug genommen wird.

### Schritt b)

Nach einer allgemeinen Vorgehensweise erfolgt die Entfernung der restlichen Säure S in Schritt b) des erfindungsgemäßen Verfahrens destillativ. Die Destillation kann bei Normaldruck oder unter vermindertem Druck erfolgen. Bevorzugt ist die Destillation unter vermindertem Druck, wobei zum Beispiel zu Beginn ein nur geringes Vakuum angelegt und dieses gegebenenfalls mit fortschreitender Destillation erhöht wird. Gewünschtenfalls ist auch eine Destillation bei einem konstanten Druck möglich. Im allgemeinen liegt der Druck in Schritt b) in einem Bereich von etwa 1 bis 100 mPa (10 bis 1000 mbar), bevorzug 1 bis 20 mPa (10 bis 200 mbar), insbesondere 1.5 bis 5 mPa (15 bis 50 mbar). Die Temperatur liegt dabei in einem Bereich von etwa 80 bis 130 °C, bevorzugt etwa 90 bis 120 °C.

Überraschenderweise wurde nun gefunden, dass der Restsäuregehalt der Ester E im allgemeinen schneller abnimmt und allgemein auch geringere Restsäuregehalte erzielt werden, wenn man in Schritt b) vor Beginn oder im Verlauf der Destillation Wasser zu dem Reaktionsgemisch gibt. Die Wasserzugabe kann dabei flüssig oder dampfförmig erfolgen. Wird flüssiges Wasser zu dem Reaktionsansatz gegeben, so erfolgt die Zugabe vorzugsweise unterhalb der Flüssigkeitsoberfläche des in Schritt a) erhaltenen Produktes, zum Beispiel mit Hilfe eines Senkrohres (Tauchrohr). Die Wasserzugabe kann dabei jeweils vollständig oder kontinuierlich nach Maßgabe des Verbrauchs zu Beginn der Destillation oder erst bei fortgeschrittener Abtrennung der Säure S und eines gegebenenfalls ebenfalls aus Schritt a) noch vorhandenen Lösungsmittels erfolgen. Vorzugsweise wird mit der Wasserzugabe erst begonnen, wenn das Lösungsmittel aus Schritt a), falls vorhanden, im wesentlichen vollständig abdestilliert ist, bzw. wenn der Reaktionsansatz einen Restsäuregehalt entsprechend einer Säurezahl von höchstens 50 mg KOH/g aufweist. Die zugegebene Wassermenge kann im allgemeinen in einem weiten Bereich variiert werden und beträgt bevorzugt 0,005 g bis 1,00 g pro Gramm an zu veresternden Komponenten aus Schritt a).

Nach einer bevorzugten Ausführungsform des erfindungsgemäßen Verfahrens gibt man vor der Zugabe des Wassers und gegebenenfalls nach Entfernung eines Teils der Säure in Schritt b) eine Lewis-Base, bevorzugt ein tertiäres Amin, wie Triethylamin, Triprokylamin oder Tributylamin, oder eine quartäre Ammoniumverbindung, wie Tetraethylammoniumbromid, Tetrapropylammoniumbromid oder Tetrabutylammoniumbromid zu dem Reaktionsansatz. Vorzugsweise wird dabei bei Einsatz einer Lewis-Base der saure Veresterungskatalysator aus Schritt a) im wesentlichen neutralisiert. Des weiteren bewirkt diese Zugabe vorteilhafterweise im allgemeinen auch eine Vermeidung der Verfärbung des resultierenden Esters E.

Zur erfindungsgemäßen Herstellung der Ester E werden α,β-monoethylenisch ungesättigte, C₃-C₆-Mono- und -Dicarbonsäuren S, die ausgewählt sind unter Acrylsäure, Methacrylsäure, Maleinsäure, Fumarsäure, Itaconsäure und Mischungen davon, eingesetzt. Bevorzugt werden als Säuren S Acrylsäure, Methacrylsäure und Mischungen davon eingesetzt.

Die zur Herstellung der Ester E eingesetzte Menge an Säure S beträgt im allgemeinen etwa 100 bis 150 Mol-% Carboxylgruppen, bezogen auf freie Hydroxylgruppen des Polykondensats P.

Geeignete Polykondensate P, die mindestens zwei freie Hydroxylgruppen pro Molekül enthalten, sind z. B. die üblichen, gegebenenfalls Ethergruppen enthaltenden Polyester bzw. gegebenenfalls Estergruppen enthaltenden Polyether. Geeignete Hydroxylgruppen-haltige Polyester können zum Beispiel in üblicher Weise durch Veresterung von zwei- oder mehrwertigen Carbonsäuren mit zweioder mehrwertigen Alkoholen hergestellt werden. Verfahren zur Herstellung von Polyestern sind dem Fachmann bekannt. Im allgemeinen werden als Carbonsäurekomponente für die Veresterung zur Herstellung von Hydroxylgruppen-haltigen Polyestern zwei- bis vierwertige C₃- bis C₃₆-Carbonsäuren, deren Ester und Anhydride eingesetzt. Dazu zählen zum Beispiel Bernsteinsäure, Bernsteinsäureanhydrid, Glutarsäure, Glutarsäureanhydrid, Adipinsäure, Sebacinsäure, Phthalsäure, Phthalsäureanhydrid, Terephthalsäure, Maleinsäure, Maleinsäureanhydrid, Fumarsäure, Citraconsäure, Tetrahydrophthalsäure, Tetrahydrophthalsäureanhydrid, Trimellithsäure, Trimellithsäureanhydrid, Pyromellithsäure und Pyromellithsäureanhydrid. Als Ausgangsstoffe für die Herstellung von Polyestern geeignete zwei- oder mehrwertige Alkohole sind zum Beispiel zwei- bis sechswertige Alkohole, z. B. Diole, wie Ethylenglykol, Propylenglykol, 1,4-Butandiol, 1,5-Pentandiol, Neopentylglykol, 1,6-Hexandiol, 2-Methyl-1,5-pentandiol, 2-Ethyl-1,4-butandiol, Dimethylolcyclohexan, Triole, wie Glycerin, Trimethylolethan, Trimethylolpropan, Trimethylolbutan, Tetraole, wie Pentaerythrit, Ditrimethylolpropan, Hexole, wie Erythrit und Sorbit. Weitere geeignete Polyesterole sind auch Polycaprolactondiole und -triole.

Weitere geeignete Hydroxylgruppen-haltige Polykondensate P sind die Alkoxilate der zuvor genannten zwei- oder mehrwertigen Alkohole. Dazu zählen zum Beispiel ethoxylierte, propoxylierte und gemischt ethoxylierte und propoxylierte zwei- bis sechswertige Alkohole und Polyesterole. Der Alkoxylierungsgrad liegt dabei in der Regel bei 1 bis 300, bevorzugt bei 2 bis 150.

Weitere geeignete Polykondensate P sind Polyalkylenglykole bzw. die Polyadditionspolymerisate von cyclischen Ethern, wie z. B. Polytetrahydrofuran. Beispiele für Polyalkylenglykole sind Polyethylenglykol, Polypropylenglykol und Polyepichlorhydrine.

Des weiteren geeignete Polykondensate P sind auch Copolymerisate, die wenigstens eine der zuvor genannten monomeren, oligomeren oder polymeren Komponenten einpolymerisiert enthalten. Dazu zählen z. B. Polyester der zuvor genannten zwei- oder mehrwertigen Carbonsäuren und Alkohole mit terminalen Carboxylgruppen oder Hydroxylgruppen und Polyetherole, wie die zuvor genannten Alkoxilate, Polyalkylenglykole und Polymerisate cyclischer Ether.

Weitere geeignete oligomere oder polymere Komponenten sind z. B. Polyurethane mit terminalen Hydroxylgruppen, Polyesterpolyurethane, die aus Polyestern als Diolkomponente und Diisocyanaten bzw. Isocyanat-terminierten Präpolymeren erhältlich sind und Polyetherpolyurethane, die aus Polyethern als Diolkomponente und den entsprechenden Diisocyanaten bzw. Isocyanat-Präpolymeren erhältlich sind.

Weitere geeignete oligomere oder polymere Komponenten sind z. B. Epoxidharze mit freien Hydroxylgruppen, wie Bisphenhol-A-diglycidylether, die z. B. mit Alkoholen, wie den zuvor genannten, zwei- und mehrwertigen Alkoholen gehärtet wurden.

Die nach dem erfindungsgemäßen Verfahren erhältlichen Ester E auf α,β-monoethylenisch ungesättigten Carbonsäuren S und Hydroxylgruppen-haltigen Polymerisaten P weisen einen Restsäuregehalt, bestimmt als Säurezahl, von höchstens 20 mg KOH/g, bevorzugt höchstens 15 mg KOH/g auf.

Die erfindungsgemäß hergestellten Ester E können zur Verarbeitung mit weiteren, zur Strahlungshärtung üblichen Reaktivverdünnern eingesetzt werden. Dazu zählen zum Beispiel 4-t.-Butylcyclohexylacrylat, Phenoxyethylacrylat, Hexandioldiacrylat, Tripropylenglykoldiacrylat, Trimethylolpropandiacrylat, sowie Acrylate von alkoxylierten Diolen und Triolen.

Die erfindungsgemäßen Ester E mit geringem Restsäuregehalt eignen sich vorzugsweise zur Herstellung von Beschichtungs- und Überzugmitteln. Diese können zweckmäßigerweise entweder durch Elektronenstrahlen oder gegebenenfalls nach Zusatz üblicher Photoinitiatoren durch UV-Strahlen vernetzt werden und ergeben Filme mit guten anwendungstechnischen Eigenschaften.

Die Erfindung wird anhand der folgenden nicht einschränkenden Beispiele näher erläutert.

### Beispiele

Die Säurezahl ist definiert als mg KOH/g Produkt und wurde ermittelt nach DIN 53402, beziehungsweise der Vorschrift des Deutschen Arzneibuchs 10 V. 3.4.1. (1993).

### Beispiel 1 (Vergleich)

38 Gew.-% 3-fach ethoxyliertes Trimethylolpropan, 37 Gew.-% Acrylsäure und 24 Gew.-% Methylcyclohexan werden in einem Kolben vorgelegt und 0,4 Gew.-% Schwefelsäure zugegeben. Die Mischung wird mit 0,21 Gew.-% Hydrochinonmonomethylether, 0,07 Gew.-% tert.-Butyl-p-kresol, 0,07 Gew.-% Triphenylphosphit, 0,07 Gew.-% Hypophosphoriger Säure (50 %ig in Wasser) und 0,004 Gew.-% Phenothiazin stabilisiert. Unter Rückfluss wird 100 % des theoretisch möglichen Wasservolumens ausgekreist, wobei ein geringer Anteil des Volumens aus Acrylsäure besteht. Danach wird vorsichtig eine Vakuum-Rampe angelegt, um ein Aufschäumen der Reaktionsmischung zu vermeiden, und Methylcyclohexan und Acrylsäure abdestilliert. Dabei steigt die Innentemperatur auf etwa 105 °C. Bei einer Säurezahl von 46,5 mg KOH/g wird 0,1 Gew.-% Tetrabutylammoniumbromid Lösung (ca. 50 %ig in Wasser) zugegeben. Es wird noch kurz unter Vakuum weiterdestilliert, um das zugegebene Wasser zu entfernen. Von diesem Ansatz wird eine Probe entnommen und unter Vakuum (3.3 MPa; 33 mbar) auf etwa 110 °C erhitzt und Acrylsäure abdestilliert. Nach 270 min ist die Säurezahl auf 15,1 mg KOH/g gesunken.

### Beispiel 2

Von dem Ansatz aus Beispiel 1 wird eine 800 g Probe nach der Tetrabutylammoniumbromid-Zugabe entnommen und unter Vakuum (3.5 mPa; 35 mbar) auf etwa 110 °C erhitzt. Wasser wird langsam über ein Senkrohr in das Harz eingetropft. Nach 225 min und 170 ml eingeleitetem Wasser ist die Säurezahl auf 3,1 mg KOH/g gesunken.

### Beispiel 3

38 Gew.-% 3-fach ethoxyliertes Trimetylolpropan, 10 Gew.-% Adipinsäure, 25,5 Gew.-% Acrylsäure und 25,5 Gew.-% Methylcyclohexan werden in einem Kolben vorgelegt und 0,4 Gew.-% Schwefelsäure zugegeben. Die Mischung wird mit 0,22 Gew.-% Hydrochinonmonomethylether, 0,7 Gew.-% tert.-Butyl-p-kresol, 0,07 Gew.-% Triphenylphosphit, 0,07 Gew.-% Hypophosphoriger Säure (50 %ig in Wasser) und 0,002 Gew.-% Phenothiazin stabilisiert. Unter Rückfluss wird 100 % des theoretisch möglichen Wasservolumens ausgekreist, wobei etwas Acrylsäure mit abgetrennt wird. Danach wird vorsichtig eine Vakuum-Rampe angelegt, um ein Aufschäumen der Reaktionsmischung zu vermeiden und Methylcyclohexan und Acrylsäure abdestilliert. Dabei steigt die Innentemperatur auf etwa 105 °C. Bei einer Säurezahl von etwa 40,7 mg KOH/g wird 0,1 Gew.-% Tributylamin zugegeben. 287 ml Wasser werden über ein Senkrohr bei 110 °C und (3 mPa) (30 mbar) über einen Zeitraum von 90 min zugegeben. Während dieser Zeit fällt die Säurezahl auf 11,3 mg KOH/g.

Wie die erfindungsgemäßen Beispiele 2 und 3 belegen, werden bei Zusatz von Wasser zur destillativen Abtrennung der Acrylsäure Produkte mit einer geringeren Säurezahl erhalten. Dabei nimmt die Säurezahl vorteilhafterweise auch schneller ab als ohne Wasserzusatz.

## Patentansprüche

1. Verfahren zur Herstellung von Estern E mit geringem Restsäuregehalt aus α,β-monoethylenisch ungesättigten C₃-C₆-Mono- und Dicarbonsäuren S und Hydroxylgruppen-haltigen Polykondensaten P, wobei man
a) die Säure S und das Polykondensat P oder die das Polykondensat P bildenden monomeren und/oder oligomeren und/oder polymeren Komponenten, gegebenenfalls in Gegenwart eines Lösungsmittels und/oder eines Veresterungskatalysators und/oder weiterer Zusatzstoffe, unter Entfernung des entstehenden Wassers umsetzt,
b) unumgesetzte Säure S destillativ entfernt,
**dadurch gekennzeichnet, dass** man zur destillativen Entfernung der Säure S in Schritt b) Wasser zugibt.

2. Verfahren nach Anspruch 1, **dadurch gekennzeichnet, dass** die Temperatur in Schritt b) 80 bis 130 °C, bevorzugt 90 bis 120 °C, beträgt.

3. Verfahren nach einem der vorhergehenden Ansprüche, **dadurch gekennzeichnet, dass** der Druck in Schritt b) 1 bis 100 mPa (10 bis 1000 mbar), bevorzugt 1 bis 20 mPa (10 bis 200 mbar), insbesondere 1.5 bis 5 mPa (15 bis 50 mbar), beträgt.

4. Verfahren nach einem der vorhergehenden Ansprüche, **dadurch gekennzeichnet, dass** man vor der Zugabe des Wassers in Schritt b) einen Teil der Säure S und/oder gegebenenfalls das Lösungsmittel aus Schritt a) destillativ entfernt.

5. Verfahren nach einem der vorhergehenden Ansprüche, **dadurch gekennzeichnet, dass** man vor der Zugabe des Wassers und gegebenenfalls nach Entfernung eines Teils der Säure S in Schritt b) eine Lewis-Base, bevorzugt ein tertiäres Amin, oder eine quartäre Ammoniumverbindung zugibt.

6. Verfahren nach einem der vorhergehenden Ansprüche, **dadurch gekennzeichnet, dass** die Umsetzung in Schritt a) in Gegenwart eines Lösungsmittels erfolgt, das mit Wasser ein azeotropes Gemisch bildet.

7. Verfahren nach Anspruch 6, **dadurch gekennzeichnet, dass** man das in Schritt a) entstehende Wasser während der Veresterung durch azeotrope Destillation entfernt.

8. Verfahren nach einem der vorhergehenden Ansprüche, **dadurch gekennzeichnet, dass** die Säure S ausgewählt ist unter Acrylsäure, Methacrylsäure, Maleinsäure, Fumarsäure, Itaconsäure und Mischungen davon.

9. Verfahren nach einem der vorhergehenden Ansprüche, **dadurch gekennzeichnet, dass** die Hydroxylzahl des Polykondensats P im Bereich von 50 bis 900, bevorzugt 300 bis 650, liegt.

10. Verfahren nach einem der vorhergehenden Ansprüche, **dadurch gekennzeichnet, dass** der Restsäuregehalt des Esters E, bestimmt als Säurezahl, höchstens 20 mg KOH/g, bevorzugt höchstens 15 mg KOH/g, beträgt.

## Claims

1. A process for preparing esters E of low residual acid content from α,β-monoethylenically unsaturated C₃-C₆ mono- and dicarboxylic acids S and hydroxyl-containing polycondensates P, where
a) the acid S and the polycondensate P or the monomeric and/or oligomeric and/or polymeric components which form the polycondensate P, are reacted in the presence or absence of a solvent and/or of an esterification catalyst and/or of further additives, with removal of the water which forms, and
b) the unreacted acid S is removed by distillation,
which comprises adding water for the distillative removal of the acid S in step b).

2. A process as claimed in claim 1, wherein step b) is carried out at from 80 to 130°C, preferably from 90 to 120°C.

3. A process as claimed in any of the preceding claims, wherein the pressure in step b) is from 1 to 100 mPa (10 to 1000 mbar), preferably from 1 to 20 mPa (10 to 200 mbar), and, in particular, from 1.5 to 5 mPa (15 to 50 mbar).

4. A process as claimed in any of the preceding claims, wherein some of the acid S and/or, if present, the solvent from step a) is removed by distillation before adding the water in step b).

5. A process as claimed in any of the preceding claims, wherein a Lewis base, preferably a tertiary amine, or a quaternary ammonium compound is added before adding the water and, if appropriate, after removing some of the acid S in step b).

6. A process as claimed in any of the preceding claims, wherein the reaction in step a) takes place in the presence of a solvent which forms an azeotropic mixture with water.

7. A process as claimed in claim 6, wherein the water formed in step a) is removed by azeotropic distillation in the course of esterification.

8. A process as claimed in any of the preceding claims, wherein the acid S is selected from acrylic, methacrylic, maleic, fumaric and itaconic acid and mixtures thereof.

9. A process as claimed in any of the preceding claims, wherein the hydroxyl number of the polycondensate P is in the range 50 to 900, preferably from 300 to 650.

10. A process as claimed in any of the preceding claims, wherein the residual acid content of the ester E, determined as the acid number, is not more than 20 mg of KOH/g, preferably not more than 15 mg of KOH/g.

## Revendications

1. Procédé de production d'esters E, présentant une faible teneur en acides résiduels mono- et dicarboxyliques S en C₃ à C₆ à insaturation α,β-monoéthylénique, et de polycondensats P contenant des groupes hydroxyle, dans lequel
a) on fait réagir l'acide S et le polycondensat P ou les monomères formant le polycondensat P et/ou les composants oligomères et/ou polymères, éventuellement en présence d'un solvant et/ou d'un catalyseur d'estérification et/ou d'autres adjuvants, en éliminant l'eau formée lors de la réaction,
b) on élimine l'acide S n'ayant pas réagi au moyen d'une distillation,
**caractérisé en ce que** l'on ajoute, dans l'étape b), de l'eau afin de réaliser l'élimination par distillation de l'acide S.

2. Procédé selon la revendication 1, **caractérisé en ce que** la température dans l'étape b) va de 80°C à 130°C, de préférence de 90°C à 120°C.

3. Procédé selon l'une quelconque des revendications précédentes, **caractérisé en ce que** la pression dans l'étape b) va de 1 mPa à 100 mPa (10 mbars à 1000 mbars), de préférence de 1 mPa à 20 mPa (10 mbars à 200 mbars), en particulier de 1,5 mPa à 5 mPa (15 mbars à 50 mbars).

4. Procédé selon l'une quelconque des revendications précédentes, **caractérisé en ce que** l'on élimine, avant l'addition de l'eau dans l'étape b), une partie de l'acide S et/ou éventuellement du solvant de l'étape a) par distillation.

5. Procédé selon l'une quelconque des revendications précédentes, **caractérisé en ce que** l'on ajoute une base de Lewis, de préférence une amine tertiaire ou un composé d'ammonium quaternaire avant l'addition de l'eau et éventuellement après l'élimination d'une partie de l'acide S dans l'étape b).

6. Procédé selon l'une quelconque des revendications précédentes, **caractérisé en ce que** l'on réalise la réaction dans l'étape a) en présence d'un solvant qui forme un mélange azéotrope avec l'eau.

7. Procédé selon la revendication 6, **caractérisé en ce que** l'on élimine l'eau formée dans l'étape a) pendant l'estérification au moyen d'une distillation azéotrope.

8. Procédé selon l'une quelconque des revendications précédentes, **caractérisé en ce que** l'on choisit l'acide S parmi l'acide acrylique, l'acide méthacrylique, l'acide maléique, l'acide fumarique, l'acide itaconique et leurs mélanges.

9. Procédé selon l'une quelconque des revendications précédentes, **caractérisé en ce que** l'indice d'hydroxyle du polycondensat P va de 50 à 900, de préférence de 300 à 650.

10. Procédé selon l'une quelconque des revendications précédentes, **caractérisé en ce que** la teneur en acides résiduels de l'ester E, déterminé en tant qu'indice d'acidité, est au maximum de 20 mg de KOH/g, de préférence au maximum de 15 mg de KOH/g.
